**Europäisches Patentamt**

**European Patent Office**

**Office européen des brevets**

(19)

(11) Numéro de publication : **0 098 193**
**B1**

(12) **FASCICULE DE BREVET EUROPÉEN**

(45) Date de publication du fascicule du brevet :
30.09.87

(51) Int. Cl.⁴ : **G 05 D 11/02, A 61 M 16/00**

(21) Numéro de dépôt : **83401204.9**

(22) Date de dépôt : **13.06.83**

(54) **Mélangeur à débitmètre pour délivrer un mélange de deux gaz dont la proportion ne peut être inférieure à une valeur déterminée.**

(30) Priorité : **29.06.82 FR 8211343**

(43) Date de publication de la demande :
**11.01.84 Bulletin 84/02**

(45) Mention de la délivrance du brevet :
**30.09.87 Bulletin 87/40**

(84) Etats contractants désignés :
**AT BE CH DE FR GB IT LI LU NL SE**

(56) Documents cités :
**DE-C- 763 642**
**FR-A- 2 203 481**
**GB-A- 1 149 767**
**GB-A- 2 041 225**
**US-A- 3 493 005**
**US-A- 4 015 617**

(73) Titulaire : **L'AIR LIQUIDE, SOCIETE ANONYME POUR L'ETUDE ET L'EXPLOITATION DES PROCEDES GEORGES CLAUDE**
**75, Quai d'Orsay**
**F-75321 Paris Cedex 07 (FR)**

(72) Inventeur : **Monnier, Jean-Pierre**
**31, avenue du Perche**
**F-78190 Maurepas (FR)**

(74) Mandataire : **Bouton Neuvy, Liliane et al**
**L'Air liquide, Société Anonyme pour L'Etude et L'Exploitation des Procédés Georges Claude 75, Quai d'Orsay**
**F-75321 Paris Cedex 07 (FR)**

## Description

L'invention concerne un mélangeur à débitmètres pour délivrer un mélange d'oxygène et de protoxyde d'azote dans des proportions déterminées, du type comportant un circuit par chacun desdits gaz, chaque circuit comportant respectivement une source de gaz, un conduit, reliant la source à un dispositif de mesure de débit, et un orifice calibré interposé dans le conduit.

Il existe certaines applications pour lesquelles la proportion minimum d'un gaz par rapport à l'autre doit être rigoureusement respectée et c'est le cas en anesthésie où la sécurité d'utilisation des mélangeurs oxygène-protoxyde d'azote est un point essentiel. Le mélange délivré doit contenir un minimum de 21 % pour ne pas être hypoxyque.

Il est connu du brevet US-A-4 015 617 un appareil mélangeur à débitmètres comportant des moyens de réglage de débit total du mélange oxygène-protoxyde d'azote associés à des moyens d'égalisation de la pression du protoxyde d'azote à celle de l'oxygène, un orifice ajustable ainsi qu'une vanne à aiguille étant prévus pour ajuster la proportion du protoxyde d'azote dans le mélange à une valeur qui ne peut être supérieure à 50 %.

La présente invention a pour but d'interdire de délivrer un mélange contenant moins de X/100 de gaz $G_1$ (oxygène) et de limiter le débit du gaz $G_2$ (protoxyde d'azote) à une valeur déterminée quel que soit le réglage du débit d'alimentation desdits gaz et propose, à cet effet, un mélangeur dans lequel chacun des circuits de gaz précités comporte un conduit relié à une source et est muni d'un orifice calibré choisi de manière que, pour une même pression P des deux gaz, les débits desdits gaz à travers ces orifices soient dans le rapport X/100 — X, des moyens étant prévus pour limiter la pression du gaz $G_2$ à celle du gaz $G_1$ ainsi que des moyens pour limiter la pression dans les deux circuits et autoriser le passage du gaz $G_1$ en dérivation de son orifice calibré à partir de ladite pression.

Le choix des orifices calibrés pour que les débits de gaz à travers ces dits orifices soient dans le rapport X/100 — X pour une même pression des deux gaz, le fait de prévoir des moyens de limitation de la pression du gaz $G_2$ à la pression du gaz $G_1$ et le fait de limiter la pression du gaz $G_1$ ont pour conséquence que le mélangeur selon l'invention délivre toujours un mélange contenant un minimum de X/100 du gaz $G_1$ et limite le débit du gaz $G_2$ à une valeur déterminée quelle que soit l'ouverture des robinets de réglage des débits.

De manière plus précise, la présente invention est caractérisée en ce que les orifices calibrés sont choisis de manière que, pour une même pression P d'oxygène et de protoxyde d'azote, le débit de protoxyde d'azote soit au plus égal à trois fois le débit d'oxygène, en ce que des moyens limiteurs de pression, disposés entre la source et l'orifice calibré, sont prévus entre les circuits d'oxygène et de protoxyde d'azote pour limiter la pression dans le circuit de protoxyde d'azote et en ce que sont également prévus des moyens pour limiter la pression dans les deux circuits à une valeur telle que le mélange délivré contient toujours au moins 25 % d'oxygène.

Selon une autre caractéristique de l'invention, les moyens de limitation précités sont constitués par un limiteur de pression muni d'une membrane commandant le positionnement d'un clapet inséré dans le conduit de protoxyde d'azote, ladite membrane étant soumise d'un côté à la pression d'oxygène et de l'autre côté à la pression du protoxyde. La pression du protoxyde d'azote ne peut alors dépasser la pression de l'oxygène.

Selon encore une autre caractéristique de l'invention, les moyens limiteurs de pression précités sont constitués par un déverseur comportant une chambre montée en dérivation dans le circuit d'oxygène et une membrane soumise à l'action d'un ressort taré prévu pour fermer et ouvrir un conduit relié au circuit d'oxygène en aval de son orifice calibré et débouchant dans la chambre précitée.

Ce déverseur s'ouvre lorsque la pression d'oxygène, fonction du débit à travers l'orifice 14 atteint la valeur déterminée par la force du ressort taré. Cette ouverture limite la pression d'oxygène et par voie de conséquence le débit de protoxyde d'azote.

D'autres caractéristiques et avantages de l'invention apparaîtront au cours de la description qui va suivre.

Dans les dessins annexés donnés uniquement à titre d'exemple :

la figure 1 montre de façon schématique un mélangeur selon l'invention applicable en particulier à l'obtention d'un mélange oxygène-protoxyde d'azote pour anesthésie ;

la figure 2 représente la caractéristique débit-pression pour l'oxygène et le protoxyde d'azote obtenue avec un mélangeur dont les deux circuits sont munis d'orifices calibrés dans le rapport des débits 25 %-75 % pour une même pression d'alimentation ;

la figure 3 présente la caractéristique débit-pression pour l'oxygène et le protoxyde d'azote obtenue avec le mélangeur de l'invention ;

les figures 4 et 5 montrent respectivement une première et une seconde variante du mélangeur de la figure 1.

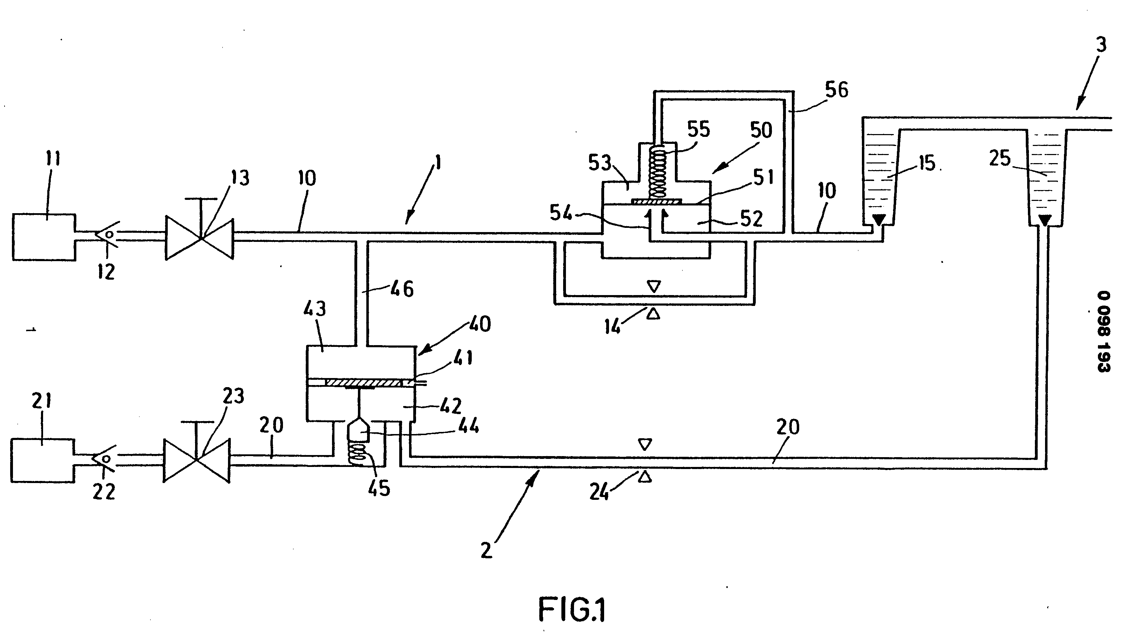

Selon le mode de réalisation représenté à la figure 1, le mélangeur comporte un circuit pour le gaz $G_1$ (oxygène), un circuit pour le gaz $G_2$ (protoxyde d'azote) et un circuit de mélange des deux gaz, ces trois circuits étant désignés d'une façon générale par 1, 2 et 3.

Le circuit 1 comporte un conduit 10 relié à une source 11 du gaz $G_1$ par l'intermédiaire d'un clapet anti-retour 12, d'une vanne 13 de réglage de débit et il comporte un orifice calibré 14 ainsi qu'un rotàmètre 15 qui sert à contrôler le débit du gaz $G_1$.

De la même façon, le circuit 2 comporte un conduit 20 relié à une source 21 du gaz $G_2$ par l'intermédiaire d'un clapet anti-retour 22, d'une vanne 23 de réglage de débit et il comporte un orifice calibré 24 et un rotàmètre 25 qui sert à contrôler le débit du gaz $G_2$.

Un limiteur de pression 40 limite éventuellement la pression du gaz $G_2$ à celle du gaz $G_1$. Le limiteur comporte une membrane 41 qui le divise intérieurement en deux chambres 42 et 43. La chambre 42 est insérée dans le conduit 20 du gaz $G_2$ et est munie d'un clapet 44 solidaire de la membrane 41 et soumise à l'action d'un ressort de rappel 45 qui tend à le pousser en position de fermeture. La chambre 43 est reliée par une canalisation 46 au conduit 10 du gaz $G_1$.

Un déverseur 50 monté en dérivation sur le conduit 10 permet de dériver le gaz $G_1$ lorsque le débit de ce dernier dépasse une valeur déterminée maintenant ainsi constante la pression dans le circuit 1 à partir de ce dit débit.

Le déverseur 50 comporte une membrane 51 qui le divise intérieurement en une chambre 52 et une chambre 53. La chambre 52 communique directement avec le circuit 10 et comporte un ajutage 54 relié audit conduit 10. Un ressort taré 55 logé dans la chambre 53 tend à appliquer la membrane 51 contre l'ajutage 54 et par conséquent à obturer ou ouvrir ce dernier selon la pression régnant dans le circuit 1.

Un conduit 56 reliant la chambre 53 du déverseur au conduit 10, en aval de la chambre 52 permet d'asservir le déverseur à la pression de sortie du gaz $G_1$ de façon à pallier les influences de pression d'utilisation en aval du mélangeur.

Dans le cas d'un mélangeur pour délivrer un mélange anesthésique oxygène-protoxyde d'azote (qui doit contenir, comme il a été indiqué plus haut au minimum de 21 % d'oxygène), l'invention se propose d'interdire de délivrer un mélange contenant moins de 25 % d'oxygène quel que soit le débit d'oxygène et de limiter le débit du protoxyde d'azote à 12 l/min.

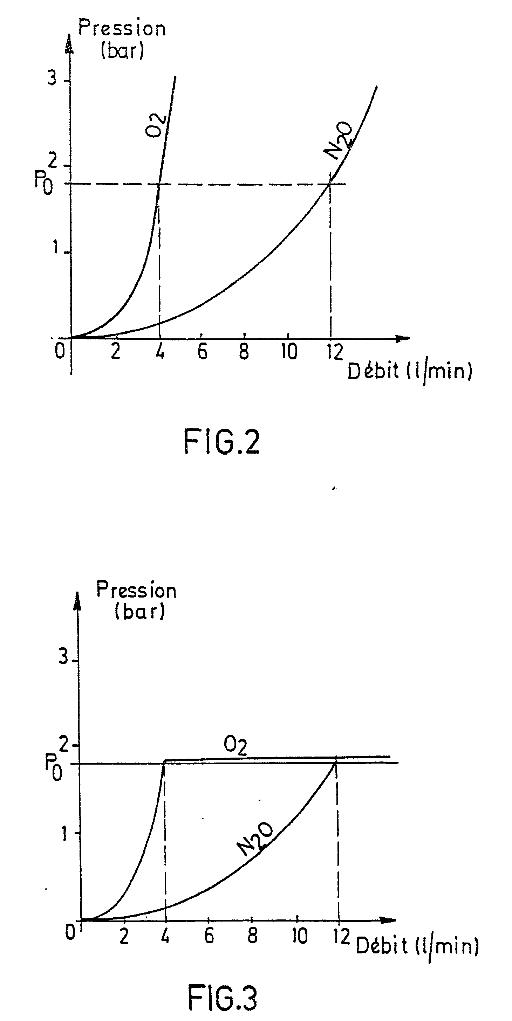

Dans ce cas, les circuits 1 et 2 de la figure 1 sont respectivement le circuit d'oxygène et le circuit du protoxyde, le circuit de mélange 3 est relié à un organe d'utilisation, par exemple un masque respiratoire (non représenté) et les orifices calibrés 14 et 24 sont choisis de façon que pour une même pression P, le débit de protoxyde d'azote soit au plus égal à trois fois le débit d'oxygène. La variation de ces débits en fonction de la pression pour de tels orifices est représentée à la figure 2.

Le détendeur 40 piloté par l'oxygène empêche que la pression du protoxyde d'azote dépasse la pression d'oxygène. Le ressort taré 55 du déverseur 50 est choisi tel que la membrane 51 dégage l'ajutage 54 lorsque le débit d'oxygène devient supérieur à 4 l/min. En conséquence, la pression dans le circuit d'oxygène, et par conséquent dans le circuit du protoxyde d'azote, est limitée à la valeur $P_0$ et le débit maximum du protoxyde d'azote est de 12 l/min comme représenté à la figure 3.

Un mélangeur, selon l'invention, peut avoir les caractéristiques suivantes :

orifice calibré 14 : diamètre = 0,45 mm permettant un débit d'oxygène de 4 l/min à 1,8 bar

orifice calibré 24 : diamètre = 0,76 mm permettant un débit de protoxyde d'azote de 12 l/min à 1,8 bar

déverseur 50 réglé à 1,8 bar de façon à limiter la pression dans les deux circuits à la valeur $P_0$ (voir figure 3) : le débit de protoxyde d'azote est ainsi limité à 12 l/min.

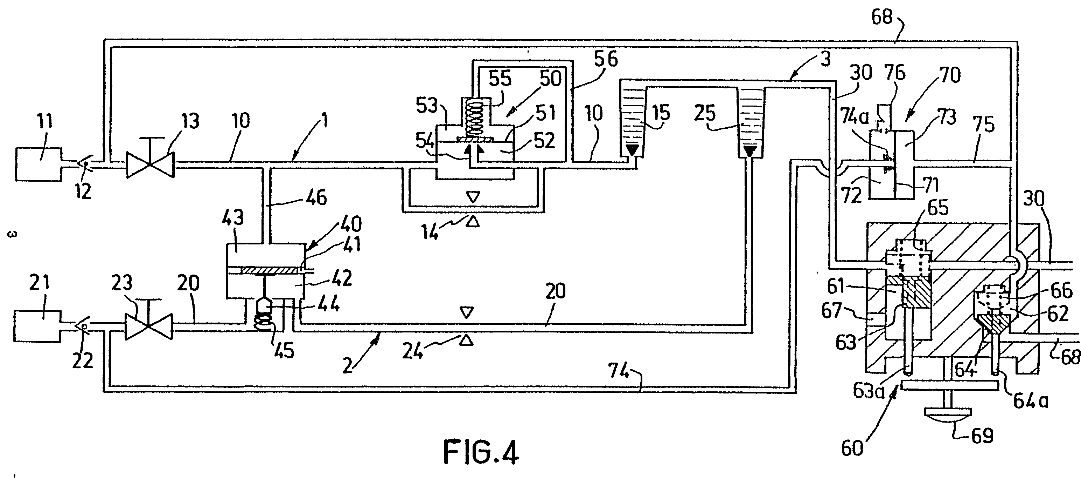

Il peut être nécessaire d'alimenter le patient non plus en le mélange oxygène-protoxyde d'azote habituel mais en oxygène pur au moins pendant un temps déterminé. On a représenté à la figure 4 une vanne d'inversion ou vanne flush 60 prévue à cet effet. La vanne 60 comporte une première et une seconde chambre 61 et 62 dans lesquelles coulissent des clapets 63 et 64 respectivement munis des tiges 63a et 64a et soumis à l'action de ressorts de rappel 65 et 66. La chambre 61 est insérée dans le conduit 30 du circuit de mélange ou circuit d'utilisation 3 et comporte un orifice 67 qui communique avec l'extérieur, tandis que la chambre 62 est insérée dans le conduit 68 relié à la source 11 d'oxygène. Un bouton-poussoir 69 agissant sur les tiges 63a, 64a permet de faire coulisser les clapets 63, 64 contre l'action des ressorts de rappel 65, 66. Les conduits 30 et 68 sont reliés à un organe d'utilisation, par exemple un masque respiratoire (non représenté). L'emplacement des conduits 30 et 68 et de l'orifice 67 dans les chambres 61 et 62 sont prévus pour assurer l'inversion de l'alimentation en mélange oxygène-protoxyde et en oxygène pur selon la position des clapets. Lorsque ceux-ci occupent la position représentée en traits pleins (position normale), l'organe d'utilisation est alimenté uniquement en mélange, lorsqu'ils occupent la position représentée en pointillés (bouton 69 enfoncé), l'organe d'utilisation est alimenté uniquement en oxygène, le mélange oxygène-protoxyde s'échappant par l'orifice 67. La vanne 60 permet donc la purge du mélange et la substitution d'oxygène pur à haut débit (supérieur à 35 litres par minute) sans qu'il y ait envoi simultané de mélange et d'oxygène.

Le mode de réalisation de la figure 4 est muni également d'un dispositif avertisseur 70 comportant une membrane 71 qui le divise intérieurement en deux chambres 72 et 73 reliées respectivement à la source 21 de protoxyde d'azote par un conduit 74 dont l'ajutage 74a est normalement en contact avec ladite membrane et à la source 11 d'oxygène par un conduit 75 lui-même relié au conduit 68 précité. Un sifflet ou analogue 76 communique avec la chambre 72. En fonctionnement normal, c'est-à-dire si la pression d'oxygène est suffisante, la membrane 71 est appliquée contre l'ajutage 74a qui est donc obturé, si la pression d'oxygène devient inférieure à une valeur déterminée (par exemple 1 bar), la membrane 71 s'écarte de l'ajutage, le protoxyde d'azote traverse le sifflet 76 qui émet un signal sonore, et avertit du manque d'oxygène. Il convient de remarquer que dans le cas où la vanne 60 est en position pour délivrer l'oxygène pur, le dispositif 70 ne fonctionne pas.

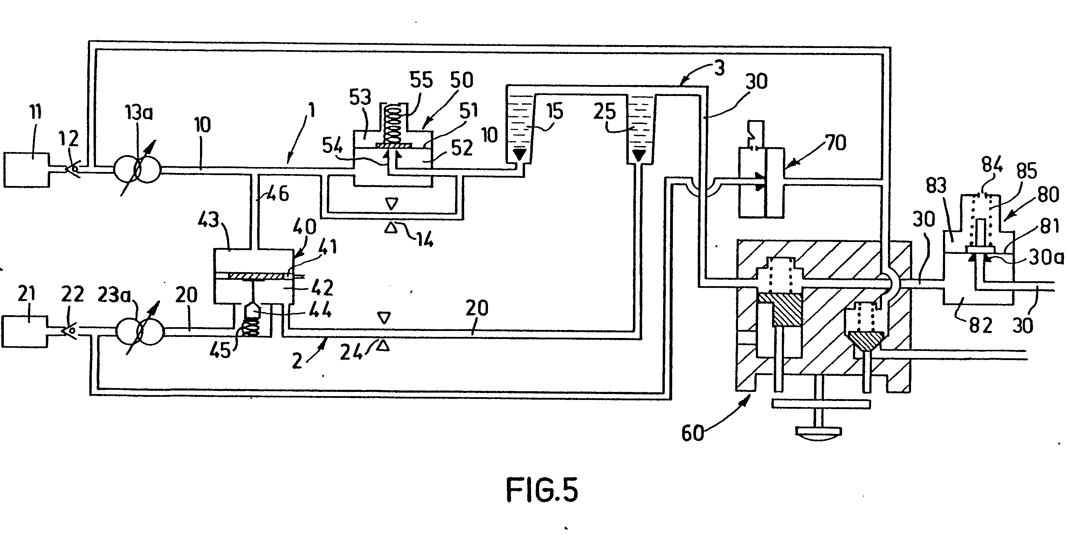

Les appareils tels que les masques respiratoires, montés dans le circuit d'utilisation, introduisent des contre-pressions qui ont pour conséquence de fausser les indications données par les débitmètres rotamétriques tels que 15 et 25, ceux-ci étant habituellement étalonnés à la pression atmosphérique. Il en résulte que le débit réel de gaz qui traverse un rotamètre peut être sensiblement différent (par exemple 1,2 fois plus élevé) que le débit lu sur ce rotamètre. Le mode de réalisation représenté à la figure 5 est muni d'un déverseur 80 monté sur le circuit d'utilisation 3, en aval de la vanne 60 donc des rotamètres 15, 25, pour pallier cet inconvénient. Le déverseur 80 comporte une membrane 81 qui le divise intérieurement en une chambre 82 et une chambre 83 communiquant avec l'extérieur par un orifice 84. La chambre 82 est insérée dans le conduit 30 du circuit 3, lequel conduit est muni d'un ajutage 30a contre lequel la membrane 81 est normalement appliquée par l'action d'un ressort 85. On étalonne le déverseur 80 au moyen du ressort 85 de façon que la pression dans les rotamètres 15, 25 soit maintenue constante quelle que soit la contre-pression dans le circuit d'utilisation. Les rotamètres doivent alors être étalonnés à la pression de tarage du déverseur (par exemple 500 m · bar) et non plus à la pression atmosphérique, et le débit qu'ils indiquent, correspond au débit réel.

Dans le mode de réalisation de la figure 5, les circuits 1 et 2 sont munis de détendeurs réglables 13a, 23a au lieu des robinets 13, 23 des modes de réalisation précédents, ces détendeurs permettant une stabilisation de la pression du débit desdits circuits quelles que soient les variations des pressions d'alimentation. La pression de détente du détendeur 23a est de préférence limitée par construction à une valeur telle que le débit de protoxyde d'azote soit limité à une valeur donnée (par exemple 12 l · m⁻¹), compte tenu de la résistance introduite par l'orifice calibré 24 et de la pression constante maintenue dans les rotamètres 15, 25.

Dans ce dernier cas, le déverseur 50, prévu sur le circuit 1, devient inutile puisqu'il a pour objet de limiter le débit du protoxyde et il peut par conséquent être supprimé.

Le déverseur 80, au lieu d'être monté en aval de la vanne 60 comme représenté à la figure 4, pourrait être monté directement en aval des rotamètres 15, 25.

Il convient de remarquer que l'utilisation des détendeurs et du déverseur sur le circuit d'utilisation permet d'obtenir un mélangeur universel, c'est-à-dire insensible aux variations de pression des sources de gaz comme aux contre-pressions des appareils d'utilisation.

On pourrait apporter au mode de réalisation décrit et représenté de nombreuses variantes sans pour autant sortir du cadre de l'invention.

**Revendications**

1. Mélangeur à débitmètres pour délivrer un mélange d'oxygène et de protoxyde d'azote dans des proportions déterminées, du type comportant un circuit par chacun desdits gaz, chaque circuit (1, 2) comportant respectivement une source de gaz (11, 21), un conduit (10, 20), reliant la source (11, 21) à un dispositif de mesure de débit (15, 25), et un orifice calibré (14, 24) interposé dans le conduit (10, 20) caractérisé en ce que les orifices calibrés (14, 24) sont choisis de manière que, pour une même pression P d'oxygène et de protoxyde d'azote, le débit de protoxyde d'azote soit au plus égal à trois fois le débit d'oxygène, en ce que des moyens limiteurs de pression (40) disposés entre la source (11, 21) et l'orifice calibré (14, 24) sont prévus entre les circuits d'oxygène et de protoxyde d'azote pour limiter la pression dans le circuit de protoxyde d'azote et en ce que sont également prévus des moyens (50 ; 13a, 23a) pour limiter la pression dans les deux circuits (1, 2) à une valeur telle que le mélange délivré contient toujours au moins 25 % d'oxygène.

2. Mélangeur à débitmètre selon la revendication 1, caractérisé en ce que les moyens pour limiter la pression des deux circuits sont constitués par un déverseur (50) comportant une chambre (52) montée en dérivation dans le circuit (1) d'oxygène et une membrane (51) soumise à l'action d'un ressort taré (55) est prévue pour obturer ou dégager un ajutage (54) relié audit circuit (1) et débouchant dans ladite chambre (52).

3. Mélangeur à débitmètre selon la revendication 2, caractérisé en ce que le déverseur (50) précité comporte une seconde chambre (53) dans laquelle est logé le ressort taré (55) précité, ladite chambre (53) étant reliée par une canalisation (56) au circuit (1) d'oxygène, en aval de ladite chambre (52).

4. Mélangeur à débitmètre selon l'une des revendications 1 à 3, caractérisé en ce qu'il comporte une vanne d'inversion (60) munie d'une première et d'une seconde chambre (61, 62) reliées respectivement au circuit d'utilisation (3) et à la source d'oxygène et dans lesquelles coulissent des clapets (63, 64) qui, selon leur position, permettent le passage du mélange et empêchent simultanément le passage de l'oxygène, ou inversement.

5. Mélangeur à débitmètre selon la revendication 4, caractérisé en ce que la vanne (60) précitée est munie d'un bouton-poussoir (69) agissant à l'encontre de ressorts de rappel (65, 66) et permettant la commande simultanée des deux clapets (63, 64).

6. Mélangeur à débitmètre selon l'une des revendications 1 à 5, caractérisé en ce qu'il comporte un dispositif avertisseur (70) relié aux sources (11, 21) d'oxygène et de protoxydes d'azote et prévu pour émettre un signal d'alarme lorsque la pression d'oxygène est inférieure à une valeur déterminée.

7. Mélangeur à débitmètre selon l'une des revendications 1 à 6, caractérisé en ce qu'il comporte un déverseur (80) inséré dans le circuit d'utilisation (3) en aval des débitmètres (15, 25) ou de la vanne (60) et muni d'un ressort taré pour compenser les contre-pressions dudit circuit d'utilisation (3).

8. Mélangeur à débitmètre selon l'une des revendications 1 à 7, caractérisé en ce que les circuits (1, 2) précités sont munis de détendeurs réglables (13a, 23a).

## Claims

1. Flow meter mixer for supplying a mixture of oxygen and nitrogen monoxide in predetermined proportions of the type comprising one circuit for each of the said gases, each circuit (1, 2) respectively comprising a gas source (11, 21), a conduit (10, 20) connecting the source (11, 21) to a device to measure the flowing through (15, 25), and a calibrated orifice (14, 24) interconnected in the conduit (10, 20), characterized in that the calibrated orifices (14, 24) are selected in such a manner, that for the same pressure P of oxygen and nitrogen monoxide the flowing through of the nitrogen monoxide is atmost equal to threetimes the flowing through of oxygen, that pressure limiting means (40) positioned between the source (11, 21) and the calibrated orifice (14, 24) are provided between the circuits of oxygen and of nitrogen monoxide for limiting the pressure in the circuit of nitrogen monoxide, and that also means (50 ; 13a, 23a) are provided to limit the pressure in the two circuits (1, 2) to such a value, that the delivered mixture always contains at least 25 percent of oxygen.

2. Flow meter mixer according to claim 1, characterized in that the means to limit the pressure of the two circuits are defined by a diverter (50) comprising a chamber (52) mounted in derivation in the circuit (1) of the oxygen and a diaphragm (51) submitted to the action of a tared spring (55) and provided to close or to release connecting tube (54) connected to the said circuit (1) and terminating in the said chamber (52).

3. Flow meter mixer according to claim 2, characterized in that the aforementioned diverter (50) comprises a second chamber (53) in which the aforementioned tared spring (55) is positioned, the said chamber (53) being connected to the circuit (1) of oxygen through a canalization (56) downstream of the said chambre (52).

4. Flow meter mixer according to one of the claims 1 to 3, characterized in that it comprises an inversion valve (60) provided with a first and a second chamber (61, 62), connected to the consumer circuit (3), respectively, and to the source of oxygen and in which flaps (63, 64) are gliding which according to their position allow the passage of the mixture and simultaneously avoid the passage of oxygen or vice versa.

5. Flow meter mixer according to claim 4, characterized in that the aforementioned valve (60) is provided with a push button (69) acting against return springs (65, 66) and permitting the simultaneous command of the two flaps (63, 64).

6. Flow meter mixer according to one of the claims 1 to 5, characterized in that it comprises an alarm device (70) connected to the sources (11, 21) of oxygen and nitrogen monoxide and provided to emit an alarm signal, when the oxygen pressure is lower than a predetermined value.

7. Flow meter mixer according to one of the claims 1 to 6, characterized in that it comprises a diverter (80) inserted in the consumer circuit (3) downstream of the flow meters (15, 25) or of the valve (60) and provided with a tared spring for compensating the counter pressures of the said consumer circuit (3).

8. Flow meter mixer according to one of the claims 1 to 7, characterized in that the aforementioned circuits (1, 2) are equipped with controllable pressure-relief valves (13a, 23a).

## Patentansprüche

1. Mischeinrichtung mit Durchflußmessern zur Lieferung eines Gemisches aus Sauerstoff und Stickstoffmonoxid in bestimmten Verhältnissen, mit einem Kreislauf für jedes der Gase, wobei jeder Kreislauf (1, 2) jeweils eine Gasquelle (11, 21), eine Leitung (10, 20), welche die Quelle (11, 21) mit einer Vorrichtung zum Messen des Durchflusses (15, 25) verbindet, und eine kalibrierte Öffnung (14, 24) aufweist, die in der Leitung (10, 20) zwischengeschaltet ist, dadurch gekennzeichnet, daß die kalibrierten Öffnungen (14, 24) derart ausgewählt sind, daß für einen gleichen Druck P an Sauerstoff und an Stickstoffmonoxid der Durchsatz an Stickstoffmonoxid höchstens gleich dem Dreifachen des Durchsatzes an Sauerstoff ist, daß zwischen der Quelle (11, 21) und der kalibrierten Öffnung (14, 24) angeordnete Druckbegrenzungsmittel (40) zwischen den Kreisläufen für Sauerstoff und für Stickstoffmonoxid vorgesehen sind, um den Druck in dem Kreislauf für Stickstoffmonoxid zu begrenzen, und daß auch Mittel (50 ; 13a, 23a) vorgesehen sind, um den Druck in den zwei Kreisläufen (1, 2) auf einen solchen Wert zu begrenzen, daß das gelieferte Gemisch immer mindestens 25 % Sauerstoff enthält.

2. Mischeinrichtung mit Durchflußmessern nach Anspruch 1, dadurch gekennzeichnet, daß die Mittel zur Begrenzung des Druckes der zwei Kreisläufe durch eine Umleitvorrichtung (50) gebildet sind, die eine Kammer (52), welche im Nebenschluß im Kreislauf (1) für Stickstoff angebracht ist, und eine Membran (51) aufweist, welche der Tätigkeit einer tarierten Feder (55) unterworfen und für das Verschließen oder Freigeben eines Ansatzrohres (54) vorgesehen ist, welches mit dem Kreislauf (1) verbunden ist und in die Kammer (52) mündet.

3. Mischeinrichtung mit Durchflußmesser nach Anspruch 2, dadurch gekennzeichnet, daß die Umleitvorrichtung (50) eine zweite Kammer (53) aufweist, in welcher die tarierte Feder (55) eingebaut ist, wobei die Kammer (53) durch ein Leitungssystem (56) mit dem Sauerstoffkreislauf (1) abstromig von der Kammer (52) verbunden ist.

4. Mischeinrichtung mit Durchflußmesser nach einem der Ansprüche 1 bis 3, dadurch gekennzeichnet, daß sie ein Umkehrventil (60) aufweist, welches mit einer ersten und einer zweiten Kammer (61, 62) versehen ist, die jeweils mit dem Verbraucherkreislauf (3) und der Sauerstoffquelle verbunden sind und in denen in einer Führung Ventile (63, 64) gleiten, die je nach ihrer Stellung den Durchfluß des Gemisches erlauben und gleichzeitig den Durchgang von Sauerstoff verhindern oder umgekehrt.

5. Mischeinrichtung mit Durchflußmesser nach Anspruch 4, dadurch gekennzeichnet, daß das Ventil (60) mit einer Drucktaste (69) ausgestattet ist, welche gegen Rückholfedern (65, 66) wirkt und die gleichzeitige Betätitung der zwei Ventile (63, 64) erlaubt.

6. Mischeinrichtung mit Durchflußmesser nach einem der Ansprüche 1 bis 5, dadurch gekennzeichnet, daß sie ein Warngerät (70) aufweist, welches mit den Quellen (11, 21) für Sauerstoff und Stickstoffmonoxid verbunden ist und dafür vorgesehen ist, ein Alarmsignal auszusenden, wenn der Sauerstoffdruck niedriger als ein vorbestimmter Wert ist.

7. Mischeinrichtung mit Durchflußmesser nach einem der Ansprüche 1 bis 6, dadurch gekennzeichnet, daß sie eine Umleitvorrichtung (80) aufweist, die in dem Verbraucherkreislauf (3) abstromig von den Durchflußmessern (15, 25) oder dem Ventil (60) eingebaut ist und mit einer karierten Feder ausgestattet ist, um die Gegendrücke des Verbraucherkreislaufes (3) auszugleichen.

8. Mischeinrichtung mit Durchflußmesser nach einem der Ansprüche 1 bis 7, dadurch gekennzeichnet, daß die Kreisläufe (1, 2) mit regelbaren Druckminderventilen (13a, 23a) ausgestattet sind.

FIG.1

FIG.2

FIG.3

2

FIG.4

0 098 193

FIG.5